# EUROPEAN PATENT SPECIFICATION

(11) **EP 2 747 690 B1**
(45) Date of publication and mention of the grant of the patent: **23.01.2019**
(21) Application number: 12753897.3
(22) Date of filing: 24.08.2012
(51) Int. Cl.: A61B 18/14, A61B 18/00

(54) **DEVICE AND METHODS FOR NERVE MODULATION**
VORRICHTUNG UND VERFAHREN ZUR NERVENMODULATION
DISPOSITIF ET PROCÉDÉS DE MODULATION DE NERFS

(30) Priority: 24.08.2011 US 201161526968 P
(43) Date of publication of application: 02.07.2014
(73) Proprietor: Boston Scientific Scimed, Inc., Maple Grove, MN 55311 (US)
(72) Inventor: SUTERMEISTER, Derek, Eden Prairie, Minnesota 55346 (US); OSTROOT, Tim A., Cokato, Minnesota 55321 (US); ANDERSON, James M., Fridley, Minnesota 55432 (US)
(74) Representative: Vossius & Partner Patentanwälte Rechtsanwälte mbB
(86) International application number: PCT/US2012/052313
(87) International publication number: WO 2013/028993

(56) References cited:
- EP-A2- 0 499 491
- WO-A1-01/19270
- US-A- 5 507 743
- US-A1- 2005 070 887
- US-A1- 2005 288 730

## Description

### Field

The disclosure generally pertains to percutaneous and intravascular devices for nerve modulation and/or ablation.

### Background

Certain treatments require the temporary or permanent interruption or modification of select nerve function. One example treatment is renal nerve ablation which is sometimes used to treat conditions related to congestive heart failure. The kidneys produce a sympathetic response to congestive heart failure, which, among other effects, increases the undesired retention of water and/or sodium. Ablating some of the nerves running to the kidneys may reduce or eliminate this sympathetic function, which may provide a corresponding reduction in the associated undesired symptoms.

Many nerves (and nervous tissue such as brain tissue), including renal nerves, run along the walls of or in close proximity to blood vessels and thus can be accessed intravascularly through the walls of the blood vessels. In some instances, it may be desirable to ablate perivascular nerves using a radio frequency (RF) electrode. In other instances, the perivascular nerves may be ablated by other means including application of thermal, ultrasonic, laser, microwave, and other related energy sources to the vessel wall.

Because the nerves are hard to visualize, treatment methods employing such energy sources have tended to apply the energy as a generally circumferential ring to ensure that the nerves are modulated. However, such a treatment may result in thermal injury to the vessel wall near the electrode and other undesirable side effects such as, but not limited to, blood damage, clotting, weakened vessel wall, and/or protein fouling of the electrode.

US 5,507,743 discloses an RF treatment apparatus for treatment of a tumor.

WO 01/19270 discloses cardiac catheter system.

EP 0 499 491 A2 discloses an endocardial mapping and/or ablation system.

US 2005/0070887 A1 discloses a probe for ablating tissue.

US 2005/0288730 A1 discloses an apparatus for renal neuromodulation.

### Summary

It is therefore desirable to provide for alternative systems and methods for intravascular nerve modulation which distribute ablation sites along and around the vessel.

The invention is defined by the appended claims. Embodiments, examples or aspects not covered by the claims are provided for a better understanding of the invention.

One illustrative embodiment of an intravascular system for nerve modulation through the wall of a blood vessel comprises an elongate member having a proximal end and a distal end, the elongate member having a radially expanding region disposed proximate the distal end; and at least one element disposed along the radially expanding region of the elongate member which is capable of ablating adjacent tissue, wherein the radially expanding region has an expanded state in which the radially expanding region forms a generally helical structure such that the at least one element capable of ablating tissue may be positioned proximate an inner wall of a blood vessel which is adjacent to the nerve to be modulated. In some embodiments, the radial expanding region may be a self-expanding region or may be expandable through a mechanical, hydro-mechanical or electrical actuation means.

Another illustrative embodiment of an intravascular system for nerve modulation through the wall of a blood vessel comprises an elongate member having a proximal end and a distal end, the elongate member having a radially expanding region disposed proximate the distal end; and a plurality of elements disposed along the radially expanding region of the elongate member which are capable of ablating adjacent tissue, wherein the radially expanding region has an expanded state in which the radially expanding region forms a generally helical structure such that the plurality of elements capable of ablating tissue may be positioned proximate an inner wall of a blood vessel which is adjacent to the nerve to be modulated, further wherein the radially expanding region has sufficient structural rigidity to maintain the generally helical structure as the expanding region is translated axially within a blood vessel while maintaining contact between the at least one element capable of ablating tissue and the vessel wall.

The disclosure also relates to a method of modulating a nerve located adjacent to a blood vessel comprising introducing a delivery sheath into a blood vessel; positioning the distal end of the delivery sheath proximate the region of the blood vessel to be treated, the delivery sheath containing a non-expanded elongate member having a radially expanding region disposed proximate a distal end thereof, the radially expanding region having generally helical form in an expanded configuration and having positioned proximate the distal end of the elongate member at least one least one element capable of ablating tissue; advancing the elongate member relative to the delivery sheath, thereby allowing the radially expanding region to expand or be expanded to a generally helical form such that the at least one element capable of ablating tissue is positioned proximate the vessel wall; activating at least one element capable of ablating tissue, thereby ablating adjacent tissue and modulating nerve tissue adjacent to the vessel; repositioning the radially expanding region of the elongate member axially while maintaining contact between the at least one element capable of ablating tissue and the vessel wall; activating at least one element capable of ablating tissue, thereby ablating adjacent tissue and modulating nerve tissue adjacent to the vessel; withdrawing the elongate member relative to the delivery sheath; and withdrawing the elongate member and delivery sheath from the body.

In some embodiments, the method includes advancing an elongate member having a radially expanding region disposed proximate the distal end, the radially expanding region having generally helical form in an expanded configuration and having positioned proximate a distal end of the elongate member a plurality of elements capable of ablating tissue; advancing the elongate member relative to a delivery sheath, thereby allowing the radially expanding region to expand to a generally helical form such that the at least some elements of the plurality of elements capable of ablating tissue are positioned proximate the vessel wall; and activating at least one element capable of ablating tissue, thereby ablating adjacent tissue and modulating nerve tissue adjacent to the vessel, wherein some of the plurality of elements capable of ablating tissue, repositioning the radially expanding region of the elongate member axially while maintaining contact between the at least some of the plurality of elements capable of ablating tissue and the vessel wall; activating at least one element capable of ablating tissue, thereby ablating adjacent tissue and modulating nerve tissue adjacent to the vessel; withdrawing the elongate member relative to the delivery sheath; and withdrawing the elongate member and delivery sheath from the body.

### Brief Description of Drawings

Fig. 1 is a schematic view illustrating a renal nerve modulation system in situ.
Fig. 2 is a schematic view illustrating the location of the renal nerves relative to the renal artery.
Figs. 3A and 3B are schematic cutaway views of an embodiment of the disclosure deployed in a blood vessel.
Fig. 4 is a schematic cutaway view of another embodiment of the disclosure deployed in a blood vessel.
Figs. 5A-C and 6 are schematic views of a portion of an embodiment of the disclosure which includes an element capable of ablating adjacent tissue.
Figs. 7A-D are cross-sectional views illustrating embodiments of renal nerve modulation systems.

### Detailed Description

The following description should be read with reference to the drawings wherein like reference numerals indicate like elements throughout the several views. The drawings, which are not necessarily to scale, depict illustrative embodiments and are not intended to limit the scope of the invention.

All numbers are herein assumed to be modified by the term "about." The recitation of numerical ranges by endpoints includes all numbers subsumed within that range (e.g., 1 to 5 includes 1, 1.5, 2, 2.75, 3, 3.80, 4, and 5).

As used in this specification and the appended claims, the singular forms "a", "an", and "the" include the plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect such feature, structure, or characteristic in connection with other embodiments whether or not explicitly described unless cleared stated to the contrary.

While the devices and methods described herein are discussed relative to renal nerve modulation through a blood vessel wall, it is contemplated that the devices and methods may be used in other applications where nerve modulation and/or ablation are desired. The term modulation refers to ablation and other techniques that may alter the function of affected nerves. When multiple ablations are desirable, they may be performed sequentially by a single ablation device mounted on an elongate member extending along a central elongate axis of the blood vessel, the elongate member having a generally helical radially self-expanding region disposed proximate the distal end wherein at least one ablation device is mounted along a generally helical portion of the elongate member.

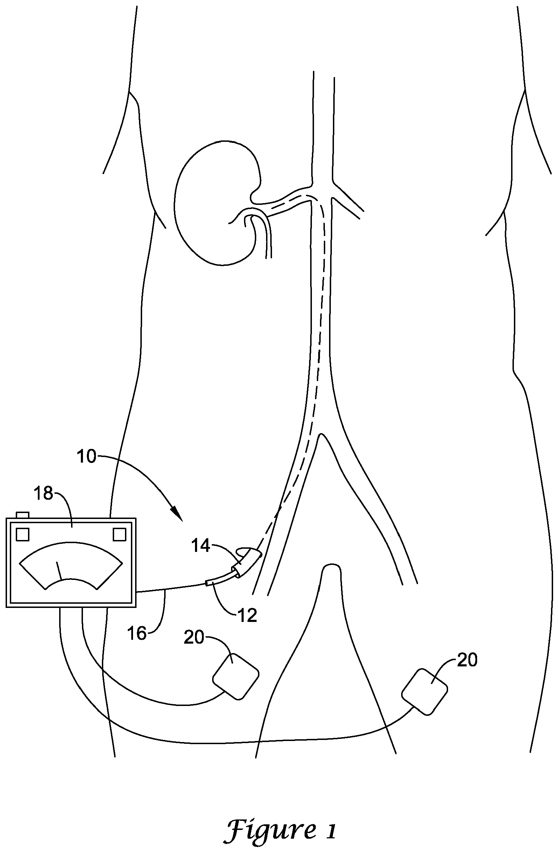

Fig. 1 is a schematic view of an illustrative renal nerve modulation system *in situ.* System 10 may include one or more conductive element(s) or wires 16 for providing power to a renal ablation system including an elongate member or shaft 12 disposed within a delivery sheath 14, which may be adapted to slidably contain the elongate member 12 when the radially expanding region (not shown) of the elongate member is in a non-expanded configuration, the details of which can be better seen in subsequent figures. A proximal end of conductive element(s) 16 may be connected to a control and power element 18, which supplies necessary electrical energy to activate one or more electrodes to which the distal end of wire(s) 16 are attached at or near a distal end of the elongate member 12. When suitably activated, the electrodes are capable of ablating adjacent tissue. The terms electrode and electrodes may be considered to be equivalent to elements capable of ablating adjacent tissue in the disclosure which follows. Suitable materials for the delivery sheath 14, elongate member 12 and elements capable of ablating adjacent tissue are known in the art and in some embodiments may include internal and/or external layers of lubricious material(s). In some instances, return electrode patches 20 may be supplied on the legs or at another conventional location on the patient's body to complete the circuit.

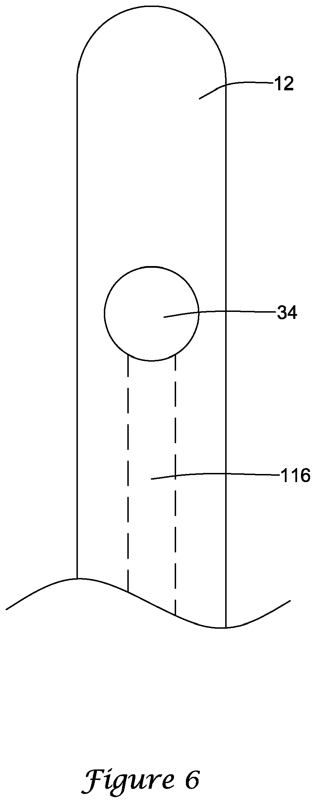

In other embodiments, element 16 of Fig. 1 may be replaced by, for example, an optical fiber 116 as in Fig. 6 to provide a conduit for laser energy rather than electrical energy. In such embodiments, the control and power element 18 may be modified appropriately.

The control and power element 18 may include monitoring elements to monitor parameters such as power, temperature, voltage, pulse size and/or shape and other suitable parameters, with sensors mounted along elongate shaft 12, as well as suitable controls for performing the desired procedure. In some embodiments, the power element 18 may control a radio frequency (RF) electrode. The electrode may be configured to operate at a frequency of approximately 460 kHz. It is contemplated that any desired frequency in the RF range may be used, for example, from 450 - 500 kHz. It is further contemplated that other ablation devices may be used as desired, for example, but not limited to resistance heating, ultrasound, microwave, and laser devices and these devices may require that power be supplied by the power element 18 in a different form.

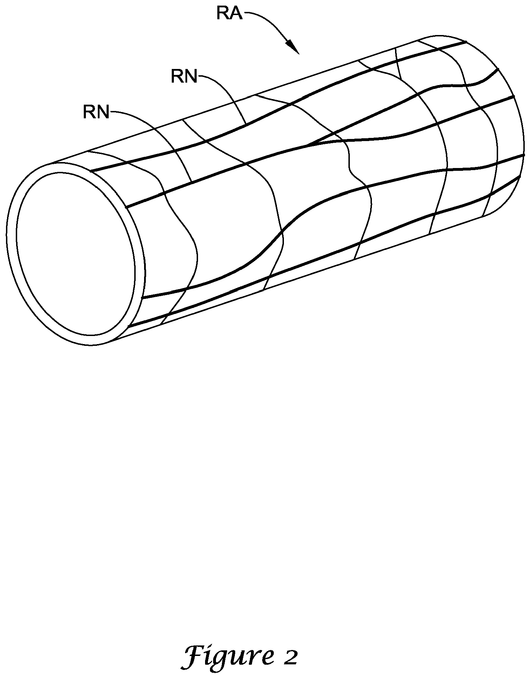

Fig. 2 illustrates a portion of the renal anatomy in greater detail. More specifically, the renal anatomy includes renal nerves RN extending longitudinally along the lengthwise dimension of renal artery RA and generally within the adventitia of the artery. As will be seen in the figure, the circumferential location of the nerves at any particular axial location may not be readily predicted. Nerves RA are difficult to visualize *in situ* and so treatment methods may desirably rely upon ablating multiple sites to ensure nerve modulation.

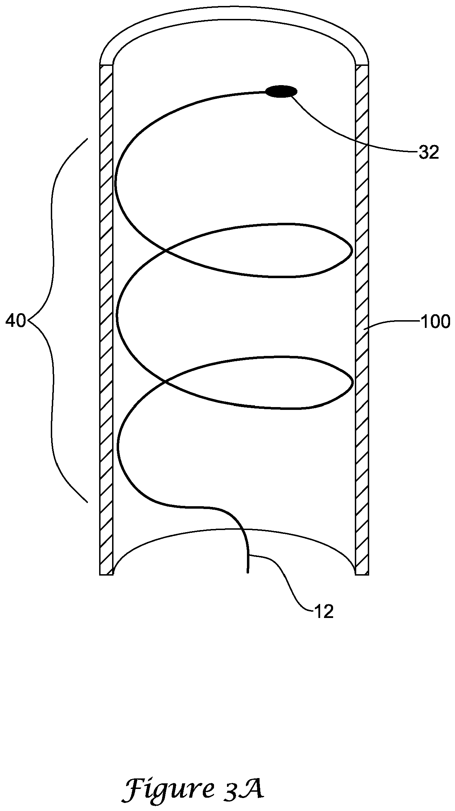

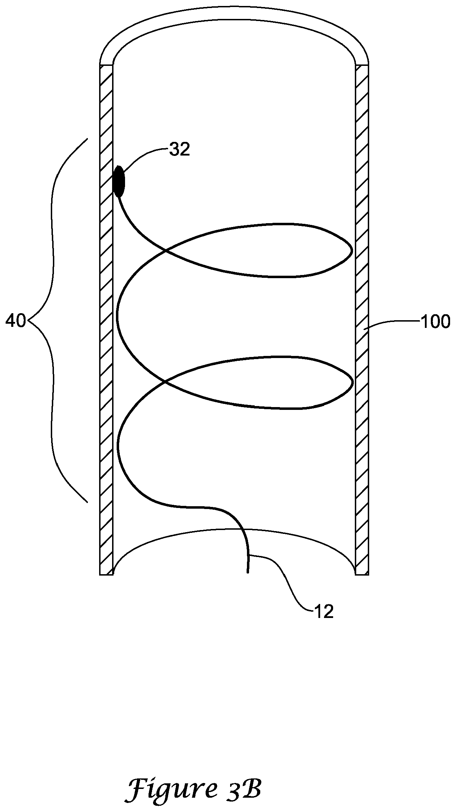

One embodiment of the distal region 40 of a renal nerve modulation device is illustrated in Figs. 3A and 3B in which an elongate shaft 12 is illustrated in an expanded configuration in which a distal region 40 of the elongate shaft 12 assumes a generally helical form when disposed along the vessel wall to be treated. For the purposes of this application, the term "generally helical" does not necessarily imply that the elongate shaft is uniformly curved throughout its distal region 40 or that it establishes continuous contact along its length with the vessel wall 100, but rather encompasses devices adapted to have a plurality of points of contact within the distal region 40 with a vessel wall in which the distal region is deployed such that the points of contact lie on a cylinder-like surface having a mean radius and an axial extent and wherein the points of contact are joined by segments of the elongated shaft. The cylinder-like surface may, of course, depart from an ideal form due to curvature or other anatomical features of the vessel in which the system is deployed. Segments within a region 40 of the elongate member 12 having a generally helical form may be radially convex outward, linear, radially concave outward, or combinations of those forms. For example, in a view axially along the vessel the distal region 40 of the elongated shaft may appear to be circular and continuously in contact with the vessel wall or it may appear to form a polygon with straight or somewhat curved segments joining points of contact. Additionally, portions of the generally helical form may be axially displaced distally and/or proximally from the helix generally defined by the points of contact with the vessel wall without departing from the spirit of the disclosure. In some embodiments, the pitch of successive turns of the helix may increase and/or decrease. The expanded generally helical distal region 40 has sufficient structural rigidity to maintain the generally helical structure as the radial expanding region is translated axially within a blood vessel while maintaining contact between the at least one element capable of ablating tissue and the vessel wall.

In this embodiment, region 40 is illustrated as a self-expanding region. That is, region 40 adopts the expanded, helical form when restraint is removed. In some embodiments, the region 40 is expandable through an actuation means (not shown) such as a pull wire, tension member, the activation of an electro-active polymer or other suitable means.

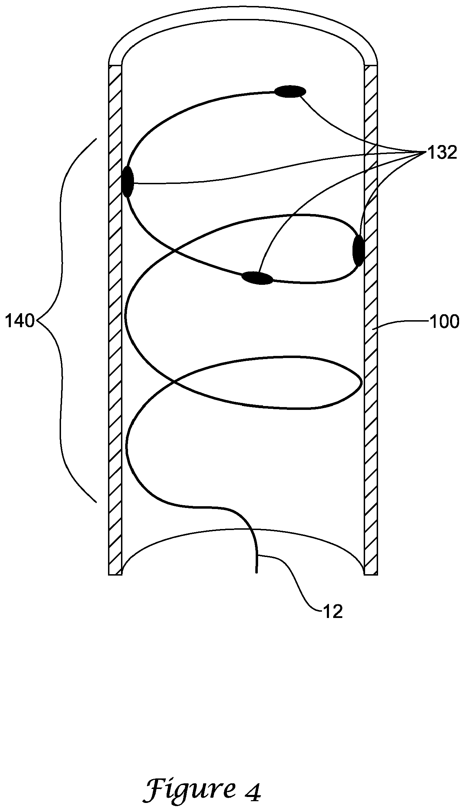

As illustrated in Figs. 3A, 3B, and 4, the elongate member 12, having been extended distally relative to the delivery sheath 14, has self-expanded to create a generally helical distal region 40 which contacts the vessel wall 100 continuously, or at a plurality of points, along the length of the generally helical distal region 40. In Figs. 3A and 3B, an element 32 (e.g., an electrode) capable of ablating tissue is disposed at the distal end of the elongate member 12. The embodiment of Fig. 4 differs from that of Figs. 3A and 3B in that the embodiment of Fig. 4 has additional elements 132 capable of ablating tissue distributed along its generally helical distal region 40. Once self-expanded, the generally helical distal region 40 has sufficient structural rigidity and/or outward directed expansion force to resist significant distortion of the helical region as the generally helical region is translated axially and/or helically within the vessel while maintaining contact between the element or elements 32, 132 capable of ablating tissue and the vessel wall 100 as illustrated in Figs. 3A and 3B in which an initially deployed generally helical region has been translated axially and rotated approximately 90 degrees. In some embodiments, at least a portion of the distal portion of the elongate member 12 proximate the generally helical distal region 40 may spiral inward to provide a smoother transition to the generally helical distal region 40 rather than subjecting the vessel wall to an abrupt outward application of force as the generally helical distal region 40 expands upon exiting the delivery sheath 14 and/or as the generally helical distal region 40 is translated within the vessel. Proximate the distal end of the elongate shaft 12 of Figs. 3A and 3B an ablation device including at least one element capable of ablating adjacent tissue, such as an RF electrode 32, is disposed with an active surface facing radially outward such that the ablation device 32 is nominally in contact with the vessel wall. In some embodiments, the at least one element capable of ablating adjacent tissue may be a resistance heating element. In other embodiments the at least one element capable of ablating adjacent tissue includes a source of laser light.

In some embodiments, the radially self-expanding region disposed proximate the distal end of the elongate member may include, disposed along the radially self-expanding region 140, a plurality of elements 132 (Fig. 4) capable of ablating adjacent tissue such that they are angularly disposed about the axis of the vessel as well as axially displaced as shown in Fig. 4. The spacing of electrodes 132 may be selected to provide multiple ablation sites with successive circumferential offsets of, for example, 30, 40, 45, 60, 70, 75, 90 degrees, or the like. Although four electrodes 132 have been illustrated with a 90 degree circumferential displacement, it will be appreciated that a greater or lesser number of electrodes may be employed and they may have other circumferential displacements. In some embodiments the electrodes 132 may provide circumferentially overlapping ablation sites while in other embodiments the ablation sites are circumferentially spaced somewhat apart.

As employed, the system 10 may, if desired, create ablation sites which overlap circumferentially due to placement of the electrodes 132 along the generally helical distal region 140 of the system and/or due to the repositioning of the system within the vessel. For example, the ablation sites which may be produced by the embodiment of Fig. 4 may be substantially reproduced by the embodiment of Fig. 3A by a succession of translations and rotations as illustrated in part in Fig. 3B. For some applications, it may be desirable to have available elongate members 12 with different nominal expanded diameters and/or nominal helical pitch(s) in the expanded configuration in order to more effectively treat nerves associated with vessels having different dimensions.

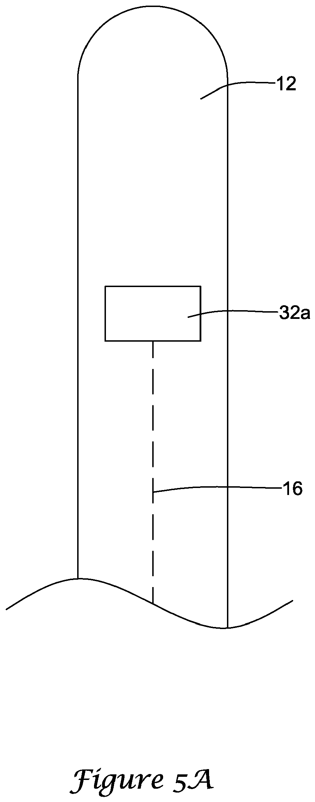

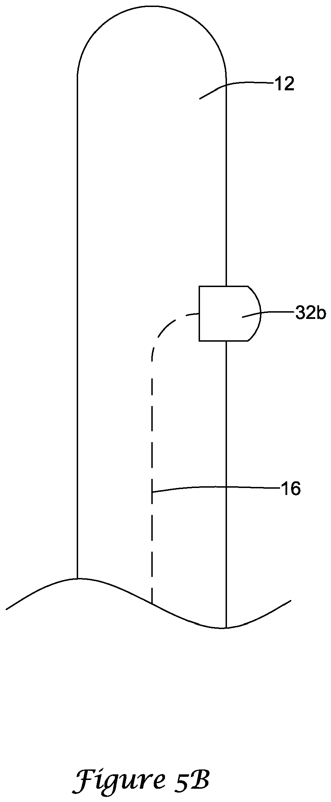

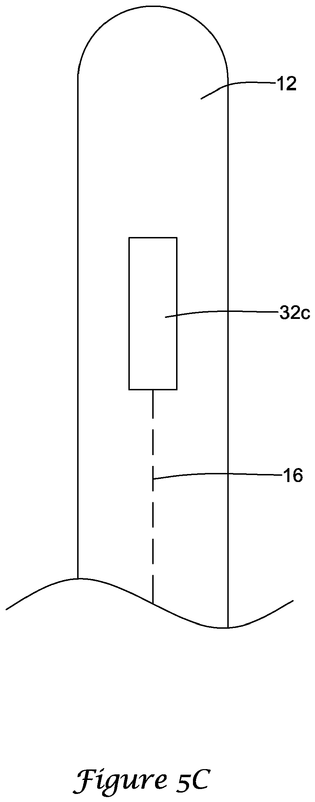

Figs. 5A-C illustrate schematically several non-limiting embodiments of electrodes suitable for use in the devices of this disclosure. Fig. 5A illustrates a substantially flush electrode 32A with a greater width than length along elongate shaft 12. Electrode 32A may be connected to power element 18 (not shown) by wire 16. Fig. 5B illustrates a side view of a similar electrode 32B which protrudes somewhat from the surface of elongate shaft 12 to ensure better contact with the adjacent vessel wall. Electrode 32B has rounded edges which may minimize mechanical damage to the vessel wall as the generally helical region in repositioned while in contact with the vessel wall and which may provide better current distribution. The electrode 32C of Fig. 5C is somewhat elongated along the elongate member 12 to allow the ablation site to subtend a somewhat longer arc circumferentially around a vessel in which the generally helical distal region 40 is deployed. Electrode 32C may also protrude from the surface of shaft 12 and may optionally include rounded edges. Similar configurations may be employed for electrodes 132.

In some embodiments, electrodes 32, 132 of the element(s) capable of ablating tissue may be formed as separate structures and attached to the elongate shaft 12. For example, electrodes 32, 132 may be machined or stamped from a monolithic piece of material and subsequently bonded or otherwise attached to the elongate shaft 12. In other embodiments, electrodes 32, 132 may be formed directly on the surface of the elongate shaft 12. For example, electrodes 32, 132 may be plated, printed, or otherwise deposited on the surface. In yet other embodiments, electrodes 32, 132 may be formed by removing portions of elongate shaft 12 which otherwise would serve as electrical insulation for wire(s) 16. In some instances, electrodes 32, 132 may be or include radiopaque marker bands. The electrodes 32, 132 may be formed from any suitable material such as, but not limited to, platinum, gold, stainless steel, cobalt alloys, or other non-oxidizing materials. In some instances, titanium, tantalum, or tungsten may be used. It is contemplated that electrodes 32, 132 may take any shape desired, such as, but not limited to, square, rectangular, circular, oblong, etc. as illustrated in Figs 5A-C. An electrode may have a length to width ratio (or width to length ratio) of between about 1:1 and 5:1, 1.5:1 to 4:1, 2:1 to 3:1 or other suitable ratio. In some embodiments, electrodes 32, 132 may have rounded edges in order to reduce the effects of sharp edges on current density. In some embodiments, the size of electrodes 32, 132 may be chosen to optimize the current density without increasing the profile of the modulation system 10. For example, an electrode 32 that is too small may generate high local current densities resulting in greater heat transfer to the blood and surrounding tissues. An electrode 32 that is too large may require a larger elongate shaft 12 to carry it.

In some embodiments, the electrodes are expandable when deployed from the delivery sheath. For example, an electrode 32 may curve to allow it to fit within the delivery sheath, and then expand to have a looser curve, a flat configuration or a curve in the opposite direction when deployed from the delivery sheath. In another example, an electrode 32 may include a resilient frame that may expand from a compressed configuration when deployed.

In some embodiments, the electrode(s) 32, 132 may extend laterally from the elongate shaft 12. In some instances, electrodes 32, 132 may have an aspect ratio of 2:1 (length to width). Such an elongated structure may provide the electrodes 32, 132 with more surface area without significantly increasing the profile of the modulation system 10. In some embodiments, electrodes 32, 132 may be a single electrode disposed around the entire perimeter of the elongate shaft 12. A single electrode 32 may allow for ablation regardless of the rotational position of elongate shaft 12 adjacent to the electrode.

It is contemplated that the system 10 may be operated in a variety of modes. In one embodiment, the system 10 may be operated in a sequential unipolar ablation mode. The ablation electrodes 132 of the elements capable of ablating tissue may each be connected to an independent power supply such that each electrode may be operated separately and current may be maintained to each electrode. In sequential unipolar ablation, one ablation electrode 132 may be activated such that the current travels from the electrode to a ground electrode (not shown) or patch(s) 20. After one ablation electrode 132 has been activated and then deactived, another ablation electrode 132 may be activated such that current travels from that ablation electrode to the ground electrode (not shown) or patch(s) 20. In another embodiment, the system 10 may be operated in a simultaneous unipolar ablation mode. In simultaneous unipolar ablation mode, the ablation electrodes 132 may be activated simultaneously such that current travels from each electrode to the ground electrode (not shown) or patch(s) 20. In some instances, the ablation electrodes 132 may each be connected to an independent electrical supply such that current may be supplied to each ablation electrode individually. In this mode, more current may be dispersed radially. This may result in a more effective, deeper penetration compared to the sequential unipolar ablation mode. In another embodiment (not shown), the system 10 may be operated in a bipolar mode. In this instance, two electrodes disposed at the treatment location may be disposed 180° from each other around the elongate shaft such that one electrode acts as the ground electrode (e.g. one cathode and one anode). As such current may flow around the elongate shaft 12 from one ablation electrode 32, 132 to the other electrode. In general, either sequential or simultaneous unipolar mode may penetrate more deeply than the bipolar mode.

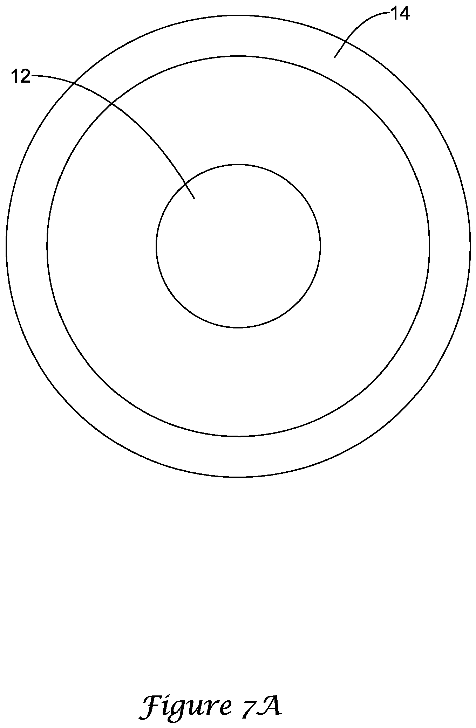

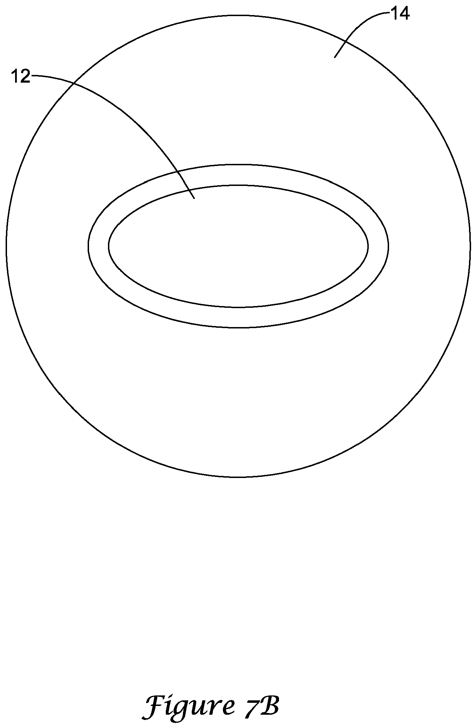

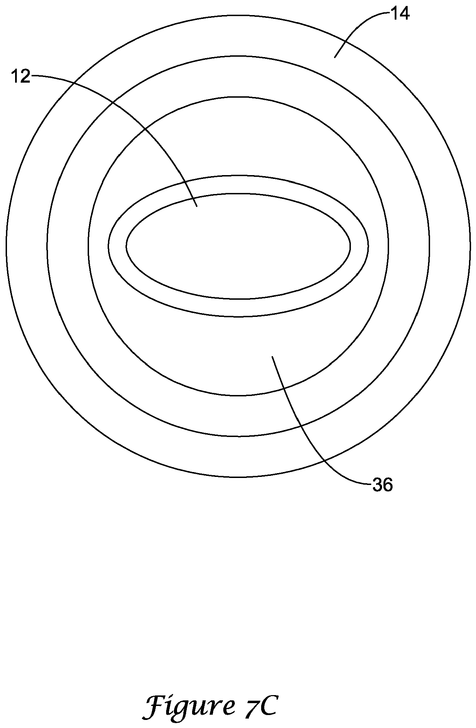

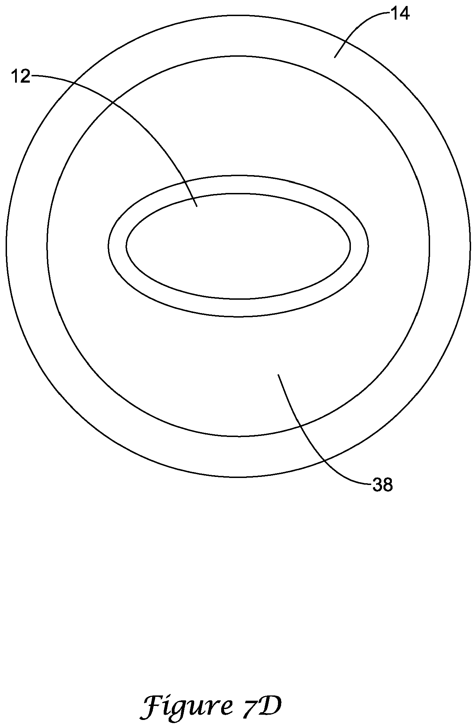

In some embodiments, the elongate member 12 may have a circular cross section, as illustrated in Fig. 7A. In such embodiments, the elongate member 12 may freely rotate within delivery sheath 14. In some embodiments, the elongate member 12 and the delivery sheath 14 may be configured to prevent relative rotation between the two elements, as illustrated in Figs. 7B-7D. It will be understood that numerous methods of preventing relative rotation of the elongate member beyond those examples illustrated herein are contemplated. In Fig. 7B, the elongate member 12 has a non-circular cross-section and the delivery sheath 14 has a corresponding non-circular lumen cross-section such that the elongate member 12 cannot be rotated within the delivery sheath. The cross-sections of the elongate member 12 and delivery sheath 14 are shown as having the same profile or shape but it can be appreciated that having a similar profile is not necessary to prevent relative rotation. The non-circular cross-sections may extend along the substantially the whole length of the elongate member 12 and/or the delivery sheath 14 or may be limited to a portion thereof. For example, the elongate member 12 may have a non-circular profile only in the distal region 40 or along the whole of the distal region 40. Fig. 7C illustrates an embodiment in which delivery sheath 14 comprises an inner sheath 36 that has the features discussed above of the delivery sheath 14 of the Fig. 7B embodiment. The inner sheath 36 may be freely slideable and rotatable within delivery sheath 14. The inner sheath 36 and the elongate member 12 are configured to prevent relative rotation as discussed above. In Fig. 7D, an insert 38 is fixed into delivery sheath 14. Insert 38 may be a disc (i.e., having a length less than its outer diameter) fixed at or proximate to the distal end of the delivery sheath or may extend for a length that is 1, 2, 3, 4 or more times the diameter of the insert or may extend for substantially the whole length of the delivery sheath.

When using an elongate member 12 having only a single element capable of ablating tissue, such as electrode 32 of Figs. 3A and 3B, the device may be repositioned axially and/or circumferentially within the vessel 100 between ablations to distribute the ablation sites along the vessel. The repositioning step may include rotation of the radially self-expanding region within the vessel 100. In other embodiments, the elongate member may have more than one ablation element capable of ablating tissue, such as electrodes 132 of Fig. 4, mounted along a generally helical portion of the elongate member 12. It also may be desirable to reposition the generally helical portion of an elongate member having multiple ablation elements, such as electrodes 132, capable of ablating tissue axially and/or circumferentially between ablations to further distribute the ablation sites along the vessel 100. The repositioning step may include rotation of the radially self-expanding region within the vessel 100. Repositioning of the generally helical distal region 40 may be accomplished by rotating a proximal end of the elongate shaft 12 to advance or retract the distal region 40 along the vessel wall 100 in the manner of a screw advancing along a threaded surface. In other instances, repositioning the generally helical distal region 40 may be accomplished by advancing or withdrawing elongate shaft 12 relative to the vessel wall 100 with or without other than incidental rotation. In some of these instances, portions of all of the elongate shaft 12 (e.g., including the helical distal region 40) may include a shape memory and/or superelectic material (e.g., a nickel-titanium alloy such as nitinol) and the helical distal region 40 and/or electrodes 32, 132 may rotate due to the shape memory and/or superelastic properties of the helical distal region 40. In other words, as the shaft 12 is proximally retracted (e.g., into the sheath 14) and/or distally advanced, the shape memory and/or superleastic properties of the helical distal region 40 may cause the helical distal region 40 and/or electrodes 32, 132 to rotate relative to the vessel wall 100.

It will be understood that nerve modulation may be carried out unilaterally or bilaterally to effect nerve modulation in one or both kidneys. Bilateral nerve modulation may require withdrawing the elongate shaft 12 within delivery sheath 14 and repositioning the delivery sheath distal end relative to the kidney before continuing treatment.

In use, a renal ablation system such as elongate member 12 may introduced percutaneously as is conventional in the intravascular medical device arts. For example, a guide wire may be introduced percutaneously through a femoral artery and navigated to a renal artery using standard radiographic techniques. A guide catheter may be introduced over the guide wire and the guide wire may be withdrawn. In some embodiments, the elongate member 12 may be introduced into the guide catheter which serves as delivery sheath 14 with the radially self-expanding region 40 compressed within the delivery sheath 14. In other embodiments, a separate delivery sheath 14 containing the elongate member 12 may be introduced into the guide catheter. Once the distal end of the delivery sheath 14 is at the desired location within the renal artery, the delivery sheath may be withdrawn to allow the radially self-expanding region 40 of the elongate shaft 12 to expand and contact the vessel wall 100. It will be understood that the elongate shaft 12 may also be extended from the delivery sheath 14 or that the delivery sheath 14 may be withdrawn as the elongate shaft 12 is advanced to effect deployment of the generally helical distal region 40 or 140. The electrodes are activated to modulate nerve tissue in any of the modes described herein. In some embodiments, the activating step includes applying RF energy to the at least one element capable of ablating tissue. In other embodiments, the activating step includes supplying electrical energy to the at least one element capable of ablating tissue. In yet other embodiments, supplying electrical energy to the at least one element capable of ablating tissue activates an ultrasonic transducer. In still further embodiments, the activating step includes supplying laser energy to the at least one element capable of ablating tissue. The system may be withdrawn following ablation.

In some embodiments, following the initial ablation, the generally helical distal region 40 or 140 may be repositioned axially while substantially maintaining contact between the one or more elements capable of ablating tissue and the vessel wall. In some instances, the generally helical distal region may also be caused to rotate relative to the vessel. In some instances, proximal withdrawal of the shaft 12 into the sheath 14 may cause the generally helical distal region 40 or 140 to be repositioned axially and radially. Following repositioning one or more additional ablations may be performed. Optionally further repositioning and ablation steps may be performed as desired.

The delivery sheath 14 may then be advanced over the elongate member 12 to compress radially self-expanding region 40 and then the delivery sheath and elongate member may be withdrawn from the patient's body. Alternatively, elongate member 12 may be withdrawn into delivery sheath 14 to compress radially self-expanding region 40 and then the delivery sheath 14 and elongate member 12 may be withdrawn from the patient's body.

Although the illustrative examples described above relate primarily to embodiments in which ablation energy is supplied to the generally helical, self-expanding distal region 40 as RF energy delivered to electrodes 32, 132 through one or more wires 16, alternate ablative energy delivery systems are also contemplated. In one such an embodiment, the ablation energy may be supplied to elements corresponding generally in location and shape to electrodes 32, 132 where it is applied to the vessel wall 100 through resistive elements or transducers as thermal energy, ultrasonic energy, microwave energy, or the like. In another embodiment, the ablation energy may be supplied in the form of laser energy delivered, for example, through an optical fiber 116 as shown in Fig. 6. As in the case of systems which employ RF electrodes, such alternate energy delivery systems may deliver energy through a single element capable of ablating tissue or through a plurality of such elements capable of ablating tissue.

## Claims

1. An intravascular system (10) for nerve modulation, comprising:
an elongate member (12) having a proximal end and a distal end, the elongate member having a radially expanding region disposed proximate the distal end; and
at least one element (32; 132) capable of ablating tissue disposed along the radially expanding region of the elongate member; and
a delivery sheath (14) adapted to slidably contain the elongate member including the radially expanding region in a non-expanded configuration;
wherein the radially expanding region has an expanded state in which the radially expanding region forms a helical structure such that the at least one element (32; 132) capable of ablating tissue is positioned along an inner wall of a blood vessel that is adjacent to a nerve to be modulated,
wherein the radially expanding region has sufficient structural rigidity to maintain the helical structure as the radially expanding region is translated axially between a first position and a second position within the blood vessel while maintaining contact between the at least one element (32; 132) capable of ablating tissue and the inner wall of the blood vessel, and
wherein the elongate member (12) and the delivery sheath (14) are configured to prevent rotation of the elongate member in the delivery sheath.

2. The intravascular system of claim 1, wherein the radially expanding region is self-expanding.

3. The intravascular system of any one of claims 1-2, wherein the at least one element (32; 132) is a single electrode proximate the distal end.

4. The intravascular system of claim 3, wherein the electrode has a length to width ratio of up to 4:1.

5. The intravascular system of any one of claims 1-4, wherein the radially expanding region includes a plurality of elements capable of ablating tissue disposed along the radially expanding region.

6. The intravascular system of claim 5, wherein the plurality of elements capable of ablating tissue are adapted to ablate tissue sequentially.

7. The intravascular system of any one of claims 1-6, wherein the elongate member (12) has a non-circular cross section.

8. The intravascular system of claim 7, wherein the radially expanding region has the non-circular cross section.

9. The intravascular system of any one of claims 1-8, wherein a distal region of the delivery sheath (14) comprises a lumen having a non-circular cross section.

10. The intravascular system of claim 9, wherein the delivery sheath (14) includes an inner sheath, and wherein the lumen having a non-circular cross section is defined within the inner sheath.

11. The intravascular system of claim 9, wherein an insert is affixed to an inner surface of the delivery sheath (14), and wherein the lumen having a non-circular cross section is defined within the insert.

12. The intravascular system of any one of claims 1-11, wherein the at least one element (32; 132) capable of ablating tissue protrudes from the elongate member (12).

13. The intravascular system of any one of claims 1-11, wherein the at least one element (32; 132) capable of ablating tissue is arranged flush with an outer surface of the elongate member (12).

## Patentansprüche

1. Intravaskuläres System (10) zur Nervenmodulation, das aufweist:
ein längliches Element (12), das ein proximales Ende und ein distales Ende aufweist, wobei das längliche Element einen radial expandierenden Bereich aufweist, der nahe zum distalen Ende angeordnet ist; und
mindestens ein Element (32; 132), das zum Abtragen von Gewebe imstande ist, das längs des radial expandierenden Bereichs des länglichen Elements angeordnet ist; und
eine Zuführungshülle (14), die eingerichtet ist, verschiebbar das längliche Element zu enthalten, das den radial expandierenden Bereich in einer nicht expandierten Konfiguration enthält;
wobei der radial expandierende Bereich einen expandierten Zustand aufweist, in dem der radial expandierende Bereich eine helikale Struktur bildet, so dass das mindestens eine Element (32; 132), das zum Abtragen von Gewebe imstande ist, längs einer Innenwand eines Blutgefäßes angeordnet ist, das zu einem Nerv benachbart ist, der moduliert werden soll,
wobei der radial expandierende Bereich eine ausreichende strukturelle Steifigkeit aufweist, um die helikale Struktur aufrechtzuerhalten, wenn der radial expandierende Bereich axial zwischen einer ersten Position und einer zweiten Position innerhalb des Blutgefäßes verschoben wird, während ein Kontakt zwischen dem mindestens einen Element (32; 132), das zum Abtragen von Gewebe imstande ist, und der Innenwand des Blutgefäßes aufrechterhalten wird, und
wobei das längliche Element (12) und die Zuführungshülle (14) konfiguriert sind, eine Drehung des länglichen Elements in der Zuführungshülle zu verhindern.

2. Intravaskuläres System nach Anspruch 1, wobei der radial expandierende Bereich selbstexpandierend ist.

3. Intravaskuläres System nach einem der Ansprüche 1 bis 2, wobei das mindestens eine Element (32; 132) eine zum distalen Ende nahe Einzelelektrode ist.

4. Intravaskuläres System nach Anspruch 3, wobei die Elektrode ein Verhältnis der Länge zur Breite von bis zu 4:1 aufweist.

5. Intravaskuläres System nach einem der Ansprüche 1 bis 4, wobei der radial expandierende Bereich mehrere Elemente aufweist, die zum Abtragen von Gewebe imstande sind, das entlang des radial expandierenden Bereichs angeordnet ist.

6. Intravaskuläres System nach Anspruch 5, wobei die mehreren Elemente, die zum Abtragen von Gewebe imstande sind, eingerichtet sind, Gewebe sequentiell abzutragen.

7. Intravaskuläres System nach einem der Ansprüche 1 bis 6, wobei das längliche Element (12) einen nicht kreisförmigen Querschnitt aufweist.

8. Intravaskuläres System nach Anspruch 7, wobei der radial expandierende Bereich den nicht kreisförmigen Querschnitt aufweist.

9. Intravaskuläres System nach einem der Ansprüche 1 bis 8, wobei ein distaler Bereich der Zuführungshülle (14) ein Lumen aufweist, das einen nicht kreisförmigen Querschnitt aufweist.

10. Intravaskuläres System nach Anspruch 9, wobei die Zuführungshülle (14) eine Innenhülle aufweist, und wobei das Lumen, das einen nicht kreisförmigen Querschnitt aufweist, in der Innenhülle definiert ist.

11. Intravaskuläres System nach Anspruch 9, wobei ein Einsatz an einer Innenfläche der Zuführungshülle (14) befestigt ist, und wobei das Lumen, das einen nicht kreisförmigen Querschnitt aufweist, im Einsatz definiert ist.

12. Intravaskuläres System nach einem der Ansprüche 1 bis 11, wobei das mindestens eine Element (32; 132), das zum Abtragen von Gewebe imstande ist, von dem länglichen Element (12) vorsteht.

13. Intravaskuläres System nach einem der Ansprüche 1 bis 11, wobei das mindestens eine Element (32; 132), das zum Abtragen von Gewebe imstande ist, bündig mit einer Außenfläche des länglichen Elements (12) angeordnet ist.

## Revendications

1. Système intravasculaire (10) pour une neuromodulation, comprenant :
un élément allongé (12) ayant une extrémité proximale et une extrémité distale, ledit élément allongé présentant une zone d'expansion radiale proche de l'extrémité distale ;
et au moins un élément (32 ; 132) permettant l'ablation d'un tissu situé le long de la zone d'expansion radiale de l'élément allongé ; et
une gaine de pose (14) prévue pour contenir de manière coulissante l'élément allongé présentant la zone d'expansion radiale dans une configuration de non-expansion ;
où la zone d'expansion radiale a un état d'expansion dans lequel la zone d'expansion radiale forme une structure hélicoïdale telle que ledit au moins un élément (32 ; 132) permettant l'ablation d'un tissu est positionné le long d'une paroi intérieure d'un vaisseau sanguin adjacent à un nerf à moduler,
où la zone d'expansion radiale a une rigidité structurelle suffisante pour maintenir la structure hélicoïdale quand la zone d'expansion radiale est translatée axialement entre une première position et une deuxième position à l'intérieur du vaisseau sanguin, un contact étant maintenu entre ledit au moins un élément (32 ; 132) permettant l'ablation d'un tissu et la paroi intérieure du vaisseau sanguin, et
où l'élément allongé (12) et la gaine de pose (14) sont prévus pour empêcher une rotation de l'élément allongé dans la gaine de pose.

2. Système intravasculaire selon la revendication 1, où la zone d'expansion radiale est auto-expansible.

3. Système intravasculaire selon la revendication 1 ou la revendication 2, où ledit au moins un élément (32 ; 132) est une électrode unique proche de l'extrémité distale.

4. Système intravasculaire selon la revendication 3, où le rapport maximal entre la longueur et la largeur de l'électrode est de 4:1.

5. Système intravasculaire selon l'une des revendications 1 à 4, où la zone d'expansion radiale comprend une pluralité d'éléments permettant l'ablation d'un tissu situé le long de la zone d'expansion radiale.

6. Système intravasculaire selon la revendication 5, où la pluralité d'éléments permettant l'ablation d'un tissu est prévue pour une ablation séquentielle du tissu.

7. Système intravasculaire selon l'une des revendications 1 à 6, où l'élément allongé (12) a une section transversale non circulaire.

8. Système intravasculaire selon la revendication 7, où la zone d'expansion radiale présente la section transversale non circulaire.

9. Système intravasculaire selon l'une des revendications 1 à 8, où une partie distale de la gaine de pose (14) comprend une lumière ayant une section transversale non circulaire.

10. Système intravasculaire selon la revendication 9, où la gaine de pose (14) comprend une gaine intérieure, et où la lumière ayant une section transversale non circulaire est définie dans la gaine intérieure.

11. Système intravasculaire selon la revendication 9, où un insert est fixé sur une surface intérieure de la gaine de pose (14), et où la lumière ayant une section transversale non circulaire est définie à l'intérieur de l'insert.

12. Système intravasculaire selon l'une des revendications 1 à 11, où ledit au moins un élément (32 ; 132) permettant l'ablation d'un tissu fait saillie de l'élément allongé (12).

13. Système intravasculaire selon l'une des revendications 1 à 11, où ledit au moins un élément (32 ; 132) permettant l'ablation d'un tissu est disposé en alignement avec une surface extérieure de l'élément allongé (12).
